Europäisches Patentamt

(19) European Patent Office

Office européen des brevets

(11) Publication number: **0 059 460 B2**

# (12) NEW EUROPEAN PATENT SPECIFICATION

(45) Date of publication of the new patent specification : **27.11.91 Bulletin 91/48**

(21) Application number : **82101507.0**

(22) Date of filing : **26.02.82**

(51) Int. Cl.⁵ : **C12P 1/04, C12N 1/20, C12N 15/00, A01N 63/00, // C12R1/07**

(54) **An insecticide and a process for its preparation.**

(30) Priority : **27.02.81 JP 28781/81**

(43) Date of publication of application :
**08.09.82 Bulletin 82/36**

(45) Publication of the grant of the patent :
**18.05.88 Bulletin 88/20**

(45) Mention of the opposition decision :
**27.11.91 Bulletin 91/48**

(84) Designated Contracting States :
**BE DE FR NL**

(56) References cited :
**GB-A- 1 106 887**
**SU-A- 268 796**
**US-A- 4 277 564**
**US-A- 4 609 550**
**US-A- 4 713 241**
**JOURNAL OF INVERTEBRATE PATHOLOGY, vol. 25, 1975, pages 355-361, Academic Press, Inc., J. NISHIITSUTSUJI-UWO et al.: "Sporeless mutants of Bacillus thuringiensis"**
**CAN. J. MICROBIOL., vol. 24, 1978, pages 492-494, A.A. YOUSTEN: "A method for the isolation of asporogenic mutants of Bacillus thuringiensis"**
**International Pest Control, Nov./Dec. 1970, pp. 25-27**

(56) References cited :
**Experientia, Vol. 31/1, Nov. 1975, pp. 1285-1287**
**Tissue & Cell, Vol. 12(2), 1980, pp.233-242**
**Mutation Research, Vol. 13, 1971, pp. 93-96**
**Bacillus thuringiensis cultures available from the US Department of Agriculture, ARM-S-30/ October 1982**
**Appl. Environ. Microbiology, June 82, pp. 1498-1500**

(73) Proprietor : **SHIONOGI & CO., LTD.**
**1-8, Doshomachi 3-chome Chuo-ku Osaka 541 (JP)**

(72) Inventor : **Wakisaka, Yoshiharu**
**4-8-7, Sakasedai**
**Takarazuka-shi Hyogo Pref. (JP)**
Inventor : **Uo, Junko**
**79, Syugakuin Nakabayashi-cho Sakyo-ku Kyoto-shi Kyoto Pref. (JP)**
Inventor : **Matsumoto, Kouichi**
**6-11, 72-401, Higashitoyonaka-cho Toyonaka-shi Osaka Pref. (JP)**
Inventor : **Ohodaira, Osamu**
**1-3-30, Takebedai**
**Tondabayashi-shi Osaka Pref. (JP)**
Inventor : **Tanaka, Kentaro**
**3-19-2, Furuedai**
**Suita-shi Osaka Pref. (JP)**

(74) Representative : **Vossius & Partner**
**Siebertstrasse 4 P.O. Box 86 07 67**
**W-8000 München 86 (DE)**

## Description

This invention relates to a process for preparing an insecticide by culturing a practically reversionless asporogenous mutant of *Bacillus thuringiensis* and to the mutant used in the process.

Chemical insecticides generally used have many disadvantages in, for example, having adverse influence on men, beasts and fish, toxic residue, induction of drug-resistant insects and the like. Many research and development programs have been carried out to find an alternative insecticide. A research for bacterial agricultural chemicals utilizing *Bacillus thuringiensis* has been made as a link in the chain of the programs and the research has been partly put to practical use. It is based on the utilization of insecticidal of activity of δ-endotoxin (abbreviated as δ-toxin hereinafter). In general, *Bacillus thuringiensis* produces crystals of δ-toxin and spores in the cells. The crystals and spores are usually released from the cells when the cell walls naturally break at the final stage of cultivation and enhance the insecticidal activity. The whole culture broth is usually dried and applied directly without separating the crystals from the spores when *Bacillus thuringiensis* is used as insecticide because the separation procedure is very complicated. Unfortunately, however, the application is extremely restricted because of the possibility that the spores in the preparation will be multiplied in nature after application and become a hazard to sericulture. The creation of an asporogenous strain of *Bacillus thuringiensis* was an attempt to eliminate this drawback by developing an insecticide containing *Bacillus thuringiensis* without adversely influencing silkworms (Jap. Pat. Pub. (unexamined) No. 50/125022).

The inventors of this invention, however, observed in their investigation that 1-10 or more spores/ml were present in a fermented broth of *Bacillus thuringiensis* asporogenous mutant discovered by the present inventors. The spores are likely to be derived from reverse mutation of the mutant. The same reverse mutation is confirmed in all reported asporogenous mutants.

In the Journal of Invertebrate Pathology 25 (1975), 355-361, the selection of five sporeless mutant strains of *Bacillus thuringiensis* after treatment with the mutagen N-methyl-N'-nitro-N-nitrosoguanidine and their insecticidal activity are described.

The inventors aimed at creating a completely reversionless asporogenous mutant which had completely lost sporulation and renaturation abilities and at developing an insecticide without adverse secondary influences on silkworms, i.e. without secondary multiplication. As the result, a practically reversionless asporogenous mutant was obtained by mutation with ethyl methanesulfonate. The inventors have completed this invention by confirming that the fermented broth of the mutant does not contain any detectable spore and is obviously utilizable as safe insecticidal substance. Thus, a reversionless asporogenous mutant was created by the present inventors for the first time. This invention provides a process for the production of a bacterial insecticide free from *Bacillus thuringiensis* spores using the said mutant in addition to a novel practically reversionless asporogenous mutant.

The above practically reversionless asporogenous mutant can be obtained by treating the spore of an asporogenous mutant with a mutagen inducing deletion mutation, e.g. alkyl alkanesulfonate, especially ethyl methanesulfonate. The parent strain to be used is asporogenous mutants of *Bacillus thuringiensis* serovar. *kurstaki*. The above-mentioned asporogenous mutants are disclosed in Jap. Pat. Pub. (unexamined) No. 50/125022, J. Invertebr. Pathol. *25*, 355-361 (1975), Eur. J. Biochem. *18*, 226-239 (1971) and Can. J. Microbiol. *24*, 492-494 (1978). An example for preparing a practically reversionless asporogenous mutant is shown below.

Experiment 1

A suspension of spores of *Bacillus thuringiensis* serovar. *kurstaki* IK (10 ml. ca. $10^8$ spores/ml, 0.1 M phosphate buffer solution, pH 7.0) was heated at 70°C for 15 minutes. After cooling to room temperature, ethyl methanesulfonate[1]) (0.25 ml) was added thereto. The mixture was shaken at 28°C for 15-18 hours and centrifuged under aseptic conditions (x4000, 20 minutes, 5°C). The precipitate was washed with a sterile saline solution by centrifugation two times and then suspended in 0.1 M sterile phosphate buffer solution (pH 7.0, 5.0 ml). The suspension was used to rub the surface of 30 Petri dishes into which CL-agar medium[2]) (15 ml) had been poured in the proportion of 100 µl of the suspension per Petri dish. After incubation at 28°C for 2-5 days, the resultant colonies were isolated about 200-300 per lot by random isolation method.

The isolated colonies were inoculated on a nutrient agar disk and after incubation at 28°C for 3-5 days the presence of spores and δ-toxin was checked. On the other hand, the colonies were incubated on CL-agar disks at 28°C for 1 day and then kept at 5°C.

In the above test, 42 colonies did not form any detectable spores and showed good δ-toxin production. They were incubated on both 10 ml of M medium[3]) and S medium[4]) at 28°C for 3-4 days under shaking. About 5 ml each of incubated media were heated at 70°C for 20 minutes and the number of spores per 1 ml were counted. The rest of the heated media was centrifuged, washed with water and lyophilized. The insecticidal activity of the thus-obtained cell on silkworms was measured. Besides, the steadiness of δ-toxin production by the test strains was also observed.

Four strains were chosen because of their properties, i.e. no formation of spores, abundant production of δ-toxin and stability. The strains were inoculated on S medium (100 ml) in Sakaguchi flasks and incubated at 28° for 3-4 days. Incubation was repeated from 7 to 17 times and the number of spores and production of δ-toxin per 5 ml of medium were measured. As a result, strain 290-1 was obtained, whose spore formation was not observed during the incubation over 17 generations.

Notes

[1]Ito, J. and J. Spizizen: Radiation Research *13*, 93-96 (1971).
[2]CL-agar medium: 0.25% glycerol, 0.5% polypeptone, 0.5% casein, 0.3% sodium chloride, 1.25% agar.
[3]M medium: 0.5% glucose, 1.0% polypeptone, 0.5% lactocasein, 1.0% cane molasses, 0.3% sodium chloride.
[4]S medium: 3.0% starch, 3.0% soybean meal, 1.5% corn steep liquor, 0.1% sodium carbonate, 0.3% ammonium sulfate.

The above strain 290-1 was named *Bacillus thuringiensis* var. *kurstaki* 290-1 and has been deposited at the Agency of Industrial Science & Technology, Fermentation Research Institute at 1-1, Higashi 1-cho, Yatabemachi, Tsukuba-gun, Ibaraki Pref. Japan under FERM-P No. 5794 since December 3, 1980. The strain has also been deposited at the American Type Culture Collection in 12301, Parklawn Drive, Rockville, Maryland, U.S.A. under accession number 31813 since February 25, 1981. Besides, in this specification the name has been corrected to *Bacillus thuringiensis* serovar. *kurstaki* 290-1 in accordance with the rules of nomenclature.

The parent strain of strain 290-1 was isolated in a test field of Shionogi & Co., Ltd. in Japan and identified to be the same as a strain HD-1 being recognized as *Bacillus thuringiensis* serovar. *kurstaki* from the description in J. Invertebr. Pathol. *11*, 335-347 (1968), ibid. *15*, 139-140 (1970), ibid. *15*, 232-239 (1970) and ibid. *22*, 273-277 (1973).

Strain 290-1 has the same properties as the parent strain except for the irreversibility of spore formation. Strain 290-1 produces almost the same amount of δ-toxin as the parent strain and does not form any spore even in 17-fold subinoculations. Additionally, no spores were detected either in 30.5 ml of S medium after 3 days incubation or in 45 ml of M medium after the same incubation period. The results of the irreversibility test of strain 290-1 are shown below.

Experiment 2

Strain 290-1 was inoculated on S medium (100 ml) in a Sakaguchi flask and incubation at 28°C for 4 days was repeated 17 times. Each time the fermented media were heated at 70°C for 15 minutes and transferred to a nutrient agar medium. After incubation at 28°C for 3-4 days, spore formation was checked. Simultaneously, the insecticidal activity was measured with the lyophilizate of the precipitate of centrifugation. The assay for measuring the activity has been disclosed by Nishiitsutsuji-Uwo et al. In J. Invertebr. Pathol. *29*, 162-169 (1977). The result is shown in Table 1.

TABLE 1

| Incubation frequency | 1 | 2 | 3 | 4 | 5 | 6 |
|---|---|---|---|---|---|---|
| Number of spores/ number of petri dishes | 0/1 | 0/5 | 0/5 | 0/5 | 0/5 | 0/2 |
| Relative activity/ml | 874 | 1036 | 645 | 1065 | 870 | 2152 |
| Incubation frequency | 7 | 8 | 9 | 10 | 11 | 12 |
| Number of spores/ number of petri dishes | 0/2 | 0/2 | 0/2 | 0/2 | 0/2 | 0/3 |
| Relative activity/ml | 1122 | 999 | 2088 | 2034 | 1533 | 733 |
| Incubation frequency | 13 | 14 | 15 | 16 | 17 | |
| Number of spores/ number of petri dishes | 0/3 | 0/3 | 0/3 | 0/3 | 0/3 | |
| Relative activity/ml | 647 | 508 | 1357 | 1164 | 1067 | |

Experiment 3

The broth of strain 290-1 preliminarily incubated on a CL-agar medium at 28°C for 1 day was inoculated on a nutrient broth and incubated at 28°C for 1 day under shaking. The fermented broth was inoculated on an M medium (60 ml) and an S medium each in a 300 ml Erlenmeyer flask at the final concentration of 2% and incubated at 28°C for 3 days. 15 ml each of the fermented media were transferred into non-necked test tubes taking care not to touch the wall and were heated at 70°C for 30 minutes. The fermented M medium was portioned into 1 ml each on 5 Petri dishes, 1.5 ml each on 20 Petri dishes and 2.0 ml each on 5 Petri dishes respectively. The fermented S medium was portioned into 0.5 ml each on 11 Petri dishes and 1.0 ml each on 25 Petri dishes respectively. All dishes were incubated at 28°C for 2.4 days and no formation of colonies was observed.

Accordingly, it was confirmed that spores were not formed in 45 ml of fermented M medium nor in 30.5 ml of fermented S medium. The result of the test was computed as ratios of spore formation to the number of living cells and to cell weight, as shown in Table 2.

TABLE 2

| | M medium | S medium |
|---|---|---|
| Number of spores/ number of living cells | $0/2.1 \times 10^{10}$ | $0/5.1 \times 10^{9}$ |
| Number of spores/ cell weight | 0/90 mg | 0/549 mg |

As shown above, the reversionless asporogenous mutant prepared by the inventors practically does not form any spores. Thus, the fermented broth itself is utilized as insecticide. Besides, the term, "insecticidal substance" as used in the present application includes not only the free crystalline δ-toxin mentioned above but also the living cells of practically reversionless asporogenous mutants of *Bacillus thuringiensis*, especially *Bacillus thuringiensis* serovar. *kurstaki* 290-1, containing the above crystals, the dead cells and the mixture, fermented broth containing bacteria cells and/or the free crystals, the condensate of the fermented broth, their dried correspondents and the like.

The production of the insecticidal substance of the above practically reversionless asporogenous strain follows the methods of producing the insecticidal substance of the parent strain. The insecticidal substance can also be produced by the method disclosed in Japan. Pat. Pub. (unexamined) No. 50/125022 and the method by Dulmage, H. T. in J. Invertebr. Pathol. *22*, 237-277 (1971). More practically, *Bacillus thuringiensis* serovar. *kurstaki* 290-1 is inoculated on an organic medium being abundant in nitrogen sources and carbon sources.

4

The yield of δ-toxin is remarkably increased when the medium contains potassium ions, the final concentration being about 2-50 mM, more favourably 3-30 mM. Additionally, production of δ-toxin is largely affected by aeration volume especially oxygen supply. The fermentation under sufficient oxygen supply brings about rich production of δ-toxin. Fermentation may be continued at about 25-30°C for about 2-5 days under highly aerobic conditions. Examples of carbon sources are sucrose, maltose, glucose, fructose, cane molasses, beet sugar, corn steep liquor and the like

Nitrogen sources are, for example, ammonium sulfate, ammonium chloride, cotton seed powder, soybean meal, casein hydrolysate and the like. Minerals and vitamins may be added, if neccessary. Fermentation is effected under aerobic condition as stated above. Submerged aeration fermentation is preferred in large scale production.

After fermentation is completed, generally used methods such as centrifugation, filtration and the like may be applied if it is preferred to isolate the insecticidal substance, such as the δ-toxin crystals, cells and the like. It is also appropriate to apply ordinary concentration and drying methods in order to pulverize the fermented broth containing cells and/or free crystals. Practically, spray-drying methods commonly used in these days can be preferably applied because fine powder can be obtained without decrease of activity. A wettable powder preparation can be obtained in one operation by adding a fixing agent, a spreader, a diluent and the like to a cell floating liquid at spray-drying. Ordinary sterilizing methods can be used, if necessary, when the product obtained by the above methods contains living cells. This can be done, for example, by heating, supersonic treatment, irradiation with X ray and the like for physical sterilization, treatment with formalin, hydrogen peroxide, sulfites, nitrofurylacrylamide, furylfuramide, chlorine compounds, β-propiolactone, nitrites, nitrates, surfactants, ethylene oxide, propylene oxide and the like for chemical sterilization and the autolysis, treatment with phage, treatment with lysozyme for biological sterilization. Heating and chemical sterilization are convenient for industrial scale production among the above sterilization methods. Spray-drying methods are particularly useful for pharmaceutical preparation without necessity for a sterilization process since the product is simultaneously heated.

The thus obtained insecticidal substance is formulated into pharmaceutical preparations, for example, tablets, granules, powders, wettable powders, suspensions, emulsions, pastes and the like. If necessary, fillers, for example, kaolin, bentonite, talc, diatomaceous earth, wheat meal and sugars, with spreaders, surfactants, stabilizers and the like may be added during the preparation procedure. Other insecticides, sterilizers, herbicides, plant growth regulators, flavours, nutrients, and the like may be added, if desired, as long as this does not produce an adverse influence on the insecticidal activity.

The insecticide obtained by the method of the present invention is effective against larvae of Lepidoptera such as *Plutella maculipennis* Curtis, *Chilo suppressalis* Walker, *Cnidocampa flavescens* Walker, *Pieris rapae crucivora* Boisduval, *Momestra brassicae* Linne, *Papilio machaon hippocrates* Felder et Felder, *Chalcosia remota* Walker, *Stauropos fagi persimilis* Butler, *Arctia caja* Linne, *Pernara guttata* Bremer et Grey and the like. The insecticide can be sprayed freely to rice fields, fields, forests and waste lands without being a hazard, to silkworms because it does not cause a secondary multiplication.

The invention is exemplified more in detail in the following examples.

Example 1

KB-5 medium: 1.0% potato starch, 0.5% glucose, 1.5% soybean meal, 2.0% Pharmamedia®, 1.0% powdery pork meat, 0.2%, polypeptone, 0.03% magnesium sulfate heptahydrate, 1.0% calcium carbonate, 0.002% zinc sulfate heptahydrate, and 0.002% ferrous chloride.

*Bacillus thuringiensis* serovar. *kurstaki* 290-1 (FERM-P No. 5794) was cultured on a nutrient agar medium at 28°C for one day, and one platinum-loopful of the resultant broth was inoculated on a nutrient medium (700 ml) in a 2L Meyer flask, and cultured with shaking (180 r.p.m.) at 28°C for 12 hours. The resultant culture broth was inoculated on a KB-5 medium (15 L) of the above mentioned composition in a 30 L jar, and after culturing under aeration and agitation (400 r.p.m.) at 28°C for 52 hours, the fermented broth was treated by sharples centrifugation (×13,000 g) to give 15.2 mg/ml (dry weight) of insecticidal material (activity: 3070/ml). The activity is measured by the method described in J. Invertebr. Pathol. 29, 162-169 (1977) (Nishiitsutsuji-Uwo, J., et al.).

Example 2

The culture was carried out in the same manner as in Example 1 employing the S medium (its composition is mentioned above) instead of the KB-5 medium for fermentation to give 14.7 mg/ml (dry weight) of insecticidal material (activity: 2279/ml).

5

Example 3

The bacterium cells obtained in Example 2 (the insoluble precipitate after centrifugation) were suspended in water so that the concentration of the insoluble component was about 5% w/v. The resultant suspension was spray-dried with Minispray® (made by Yamato Scientific Co. Ltd., model DL-21). Minispray® is maintained under the following conditions: inlet temperature: 190°C; outlet temperature: 90°C or lower; flow rate of solution: 3-4 ml/min.; flow rate of hot air: 0.4-0.5 m³/min.; spraying pressure at the binary nozzle: 3.0-1.0 Kg/cm; air rate at the nozzle: 13-9 L/min.

The activity and particle size of the resultant powder specimen are shown in Table 3 in comparison with the result of a powder specimen prepared by lyophilization of part of the above suspension followed by pulverization with a fluid energy mill (alias jet mill). Powder specimens prepared by the above two methods respectively are almost equal in activity and particle size.

## TABLE 3

| The spray-drying method | | | | | | The jet mill method[1] |
|---|---|---|---|---|---|---|
| **A. Binary nozzle** | | | | | | |
| Spraying pressure kg/cm² | 3.0 | 2.5 | 2.0 | 1.5 | 1.0 | |
| Air rate L/min. | 13 | 13 | 11 | 11 | 9 | |
| **B. Dried powder** | | | | | | |
| Moisture % | 8.7 | 7.3 | 6.9 | 4.9 | 6.2 | 5.5 |
| $LC_{50}$[2] µg/ml | 87 | 59 | 66 | 71 | 71 | 58 |
| **Particle size (R) %[3]** | | | | | | |
| > 10 µ | 8.6 | 8.8 | 6.9 | 8.4 | 8.9 | 6.0 |
| > 4 µ | 13.5 | 14.0 | 12.5 | 14.4 | 13.9 | 12.0 |
| > 2 µ | 25.6 | 26.3 | 23.8 | 28.0 | 26.8 | 24.7 |
| > 1.5 µ | 39.7 | 40.0 | 35.0 | 43.3 | 39.7 | 33.7 |
| > 1.2 µ | 53.7 | 52.5 | 49.0 | 56.3 | 54.3 | 49.5 |
| > 1.0 µ | 64.2 | 64.0 | 60.7 | 66.2 | 65.8 | 54.7 |
| > 0.8 µ | 75.2 | 75.3 | 73.2 | 76.8 | 76.4 | 66.4 |
| > 0.6 µ | 85.5 | 85.4 | 84.0 | 86.5 | 85.3 | 79.0 |

Note
[1] The lyophilizate contained 2.7% moisture and the activity was $LC_{50}$ 67 µg/ml.
[2] $LC_{50}$ shows 50% lethal concentration (µg/ml) for silkworms.
[3] Particle size was measured with an apparatus (CP-50; Shimazu Co., Ltd.) of phototransmission type for measuring particle size distribution on the basis of centrifugal sedimentation.

Example 4

In the same manner as in Example 2, the culture was performed under aeration and agitation on the S medium for 54 hours to give insecticidal material specimens. The resultant insecticidal specimens were suspended in water so that the concentration of the insoluble solid component was about 3%. The additives as shown in Table corresponding to one third amount of the insoluble solid component were added to the suspension and spray-dried. The calculated $LD_{50}$ value in this case is 86.

The activity of the obtained specimens and the effect of sterilization are shown in Table 4.

TABLE 4

| Sample No. | Additive | $LC_{50}$ µg/ml | Number of live bacteria/mg |
|---|---|---|---|
| 1 | None | 53 | 0 |
| 2 | None | 45 | 0 |
| 3 | Gum arabic powder | 62 | 0 |
| 4 | Methyl cellulose (500 cps) | 57 | 0 |
| 5 | Polyvinylalcohol | 75 | 0 |
| 6 | Carboxymethyl cellulose | 78 | 0 |
| 7 | Hydroxypropylmethyl cellulose phthalate (pH 5.0) | 84 | 0 |
| 8 | New Cargen NX 405H*® | 72 | 0 |
| 9 | Talc | 70 | 0 |
| | Lyophilized specimen | 65 | — |

*Made by Takemoto Oil Fat Co. Ltd.

Example 5

A powdery preparation is prepared by combining spray-dry powder (25 parts) from *Bacillus thuringiensis* var. *kurstaki* 290-1 obtained in Example 3 with talc (75 parts). The preparation may be used in an amount of 100 g or more per 10 are.

Example 6

A spray-dry powder (25 parts) obtained in Example 3, sodium dodecylbenzenesulfonate (2 parts), sodium dinaphthylmethanedisulfonate (2 parts) and a mixture (71 parts) of kieselguhr and clay are mixed and pulverized to give wettable powder.

Example 7

A powdery preparation is prepared by combining the powder (30 parts) obtained by spray-drying together with carboxymethyl cellulose as in Example 4 with talc (70 parts).

**Claims**

1. A process for preparing an insecticide being free from Bacillus thuringiensis spores which comprises cultivating the asporogenous mutant Bacillus thuringiensis serovar. kurstaki 290-1 (ATCC No. 31813) being a mutant which does not reverse to form any detectable spores after at least 17-fold subinoculations and collecting the produced insecticidal substance.

2. The process claimed in claim 1 wherein the collection is effected by spray-drying the fermented broth.

3. Bacillus thuringiensis serovar. kurstaki 290-1 (ATCC No. 31813).

**Patentansprüche**

1. Verfahren zur Herstellung eines von Bacillus thuringiensis-Sporen freien Insektizides, umfassend die Züchtung der sporenlosen Mutante Bacillus thuringiensis serovar kurstaki 290-1 (ATCC Nr. 31813), die nach

7

mindestens 17-facher Subinoculation nicht unter Bildung irgendwelcher nachweisbaren Sporen revertiert, und die Gewinnung der erzeugten, insektiziden Substanz.

2. Verfahren nach Anspruch 1, bei dem die Gewinnung durch Sprühtrocknung des fermentierten Kulturmediums ausgeführt wird.

3. Bacillus thuringiensis serovar. kurstaki 290-01 (ATCC Nr. 31813).

## Revendications

1. Procédé de préparation d'un insecticide exempt de spores de Bacillus thuringiensis, qui comprend la culture du mutant asporogène Bacillus thuringiensis serovar. kurstaki 290- 1 (ATCC n° 31813), lequel mutant est sans réversion pour former d'éventuelles spores décelables après au moins 17 subinoculations, et la récolte de la substance insecticide produite.

2. Procédé suivant la revendication 1, caractérisé en ce que la récolte est effectuée par séchage par pulvérisation du bouillon fermenté.

3. Le Bacillus thuringiensis serovar. kurstaki 290-1 (ATCC n° 31813).